(19)

Europäisches
Patentamt
European
Patent Office
Office européen
des brevets

(11) **EP 4 317 261 A1**

(12) **EUROPEAN PATENT APPLICATION**
published in accordance with Art. 153(4) EPC

(43) Date of publication:
**07.02.2024 Bulletin 2024/06**

(21) Application number: **22779761.0**

(22) Date of filing: **01.03.2022**

(51) International Patent Classification (IPC):
**C08J 3/12** $^{(2006.01)}$

(52) Cooperative Patent Classification (CPC):
**C08J 3/12**

(86) International application number:
**PCT/JP2022/008693**

(87) International publication number:
**WO 2022/209536 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.03.2021 JP 2021056857**

(71) Applicant: **Sumitomo Seika Chemicals Co., Ltd.
Kako-gun, Hyogo 675-0145 (JP)**

(72) Inventors:
- **YAMAMOTO, Tomoka
  Himeji-shi, Hyogo 672-8076 (JP)**
- **SAWAKI, Hiroki
  Himeji-shi, Hyogo 672-8076 (JP)**

(74) Representative: **Vossius & Partner
Patentanwälte Rechtsanwälte mbB
Siebertstrasse 3
81675 München (DE)**

(54) **METHOD FOR PRODUCING WATER-ABSORBING RESIN PARTICLES**

(57) One aspect of the present invention is a method for producing water-absorbent resin particles including forming a coating layer on at least part of a surface of water-absorbent polymer particles to obtain coated resin particles; and heat-treating the coated resin particles, at a temperature higher than a glass transition temperature of a polymer component included in the coating layer and lower than a 10% weight-loss temperature, to obtain water-absorbent resin particles, in which the absolute value for the amount of change in the elongation rate of the polymer component before and after the heat treatment is 30% or more and 500% or less.

EP 4 317 261 A1

## Description

### Technical Field

The present invention relates to a method for producing water-absorbent resin particles.

### Background Art

Water-absorbent resin particles are widely used in various fields such as sanitary materials such as disposable diapers, sanitary products, and portable toilets, agricultural and horticultural materials such as water retention agents and soil conditioners, and industrial materials such as water sealants and anti-condensation agents. For water-absorbent resin particles, there is a demand to control the water absorption rate in addition to performances such as high water-absorbent ability and gel strength. It is possible to control the water absorption rate by varying the specific surface area of the water-absorbent resin particles and the use amount of a crosslinking agent. For example, Patent Literature 1 indicates that, by subjecting a hydrogel-like substance having an internal crosslinked structure to a post-crosslinking reaction, the crosslinking density is increased in the vicinity of the surface of a water-absorbent resin, thereby increasing the water absorption rate.

### Citation List

### Patent Literature

[Patent Literature 1] JP 2016-28117 A

### Summary of Invention

### Technical Problem

Upon coming into contact with a liquid to be absorbed (water, urine, or the like), water-absorbent resin particles start absorbing the liquid and enter a swollen state (a state where it is not possible to absorb any more liquid) in a short period of time. Therefore, uses of the water-absorbent resin particles are limited in fields where there is a demand to start water absorption after a certain period of time after coming into contact with a liquid or to not immediately absorb a large amount of a liquid. Accordingly, if it becomes possible to control the water absorption behavior of water-absorbent resin particles, the fields in which the water-absorbent resin particles may be used will expand.

An object of the present invention is to provide a method for producing water-absorbent resin particles that can be controlled to slow the water absorption rate.

### Solution to Problem

A method for producing water-absorbent resin particles according to the present invention includes forming a coating layer on at least part of a surface of water-absorbent polymer particles to obtain coated resin particles, and heat-treating the coated resin particles, at a temperature higher than a glass transition temperature of a polymer component included in the coating layer and lower than a 10% weight-loss temperature, to obtain water-absorbent resin particles, in which an absolute value for an amount of change in an elongation rate of the polymer component before and after the heat treatment is 30% or more and 500% or less.

### Advantageous Effects of Invention

According to the present invention, it is possible to provide a method for producing water-absorbent resin particles that can be controlled to slow the water absorption rate.

### Description of Embodiments

A detailed description will be given below of embodiments of the present invention. However, the present invention is not limited to the following embodiments and it is possible to make various modifications within the scope of the gist thereof.

In the present specification, "acrylic" and "methacrylic" are collectively denoted as "(meth)acrylic". "Acrylate" and "methacrylate" are also similarly denoted as "(meth)acrylate". "(Poly)" means both with and without the "poly" prefix.

In the numerical ranges described stepwise in the present specification, it is possible to arbitrarily combine the upper limit value or lower limit value of the numerical range in a particular step with the upper limit value or lower limit value of the numerical range in another step. In the numerical ranges described in the present specification, the upper limit value or lower limit value of the numerical ranges may be replaced with the values shown in the Examples. The materials exemplified in the present specification may be used alone or in a combination of two or more. In a case where there are a plurality of substances corresponding to each component present in the composition, the content of each component in the composition means the total amount of the plurality of substances present in the composition unless otherwise specified. "Room temperature" means $25 \pm 2$°C. In addition to shape structures formed over the entire surface, the term "layer" also encompasses shape structures which are partially formed when observed in a plan view.

The method for producing water-absorbent resin particles according to the present embodiment includes forming a coating layer on at least part of a surface of water-absorbent polymer particles to obtain coated resin particles, and heat-treating the coated resin particles, at a temperature higher than a glass transition temperature of a polymer component included in the coating layer and lower than a 10% weight-loss temperature, to obtain water-absorbent resin particles, in which an absolute value for an amount of change in an elongation rate of the polymer component before and after the heat treatment is 30% or more and 500% or less.

According to the production method according to the present embodiment, it is possible to suppress the water absorption rate of the water-absorbent resin particles by heat-treating the coated resin particles having a coating layer under predetermined conditions. Changing the elongation rate of the polymer component included in the coating layer before and after the heat treatment makes it possible to suppress the water absorption behavior. The water-absorbent resin particles according to the present embodiment have a coating layer coated with a polymer component for which an absolute value of the amount of change in the elongation rate due to the heat treatment is 30% or more and 500% or less, which makes greater control of the water absorption rate possible. The present inventors presume that, by using a polymer component for which the elongation rate after heating is +30% or more than the elongation rate before heating, the coating layer is easily deformed by a heat treatment and it is possible to reduce defects in the coating layer while, by using a polymer component for which the elongation rate after heating is -30% or less than the elongation rate before heating, the coating layer is not easily deformed by a heat treatment and it is possible to suppress swelling of the polymer particles.

The absolute value of the amount of change in the elongation rate of the polymer component is the difference between the elongation rate of the polymer film produced from the polymer component before heat treatment and the elongation rate after heat treatment. Since it is easy to control the absolute value of the amount of change in the elongation rate in the polymer component such that the water absorption rate is slowed down, the absolute value may be 32% or more, 34% or more, 36% or more, 40% or more, 50% or more, 60% or more, 70% or more, 80% or more, 100% or more, or 120% or more, and may be 400% or less, 350% or less, 300% or less, or 280% or less.

In a case where the amount of change in the elongation rate in the polymer component is a positive value, from the viewpoint that the coating layer is more easily deformed by a heat treatment and it is possible to reduce defects in the coating layer, the amount of change in the elongation rate is in a range of +30% to +500% and may be +40% or more, +50% or more, +70% or more, or +80% or more and +400% or less, +300% or less, +200% or less, or +150% or less.

In a case where the amount of change in the elongation rate in the polymer component is a negative value, from the viewpoint that the coating layer is less easily deformed by a heat treatment and it is possible to suppress swelling of the polymer particles, the amount of change in the elongation rate is in a range of -500% to -30% and may be -400% or more, -300% or more, -280% or more, or -200% or more and -35% or less, - 40% or less, -50% or less, or -60% or less.

It is possible to produce the coated resin particles by mixing polymer particles and a coating material. The polymer particles (object to be coated) according to the present embodiment are not particularly limited other than being formed of a resin having a water-absorbent property. The polymer particles may include, for example, crosslinking polymers formed by polymerization of monomers including ethylenically unsaturated monomers. It is possible for the crosslinking polymer to have monomer units derived from ethylenically unsaturated monomers. It is possible to produce polymer particles, for example, using a method including a step for polymerizing a monomer including an ethylenically unsaturated monomer. Examples of the polymerization method include a reverse phase suspension polymerization method, an aqueous solution polymerization method, a bulk polymerization method, a precipitation polymerization method, and the like.

The ethylenically unsaturated monomer may be a water-soluble ethylenically unsaturated monomer (an ethylenically unsaturated monomer for which the solubility in 100 g of ion-exchanged water is 1.0 g or more at 25°C). Examples of water-soluble ethylenically unsaturated monomers include (meth)acrylic acid and salts thereof, 2-(meth)acrylamide-2-methylpropanesulfonic acid and salts thereof, (meth)acrylamide, N,N-dimethyl(meth)acrylamide, 2-hydroxyethyl(meth)acrylate, N-methylol(meth)acrylamide, polyethylene glycol mono(meth)acrylate, N,N-diethylaminoethyl(meth)acrylate, N,N-diethylaminopropyl(meth)acrylate, and diethylaminopropyl(meth)acrylamide. In a case where the ethylenically unsaturated monomer has an amino group, the amino group may be quaternized. Ethylenically unsaturated monomers may be used alone or in a combination of two or more.

In a case where the ethylenically unsaturated monomer has an acid group, the acid group may be neutralized using an alkaline neutralizing agent before being used in the polymerization reaction. The neutralization degree in the ethylenically unsaturated monomer using an alkaline neutralizing agent is, for example, 10 mol% to 100 mol%, 50 mol% to 90 mol%, or 60 mol% to 80 mol% of the acid groups in the ethylenically unsaturated monomer.

From the viewpoint of the ease of industrial availability, the ethylenically unsaturated monomer may include at least one compound selected from the group consisting of (meth)acrylic acid and salts thereof, acrylamide, methacrylamide, and N,N-dimethylacrylamide. The ethylenically unsaturated monomer may include at least one compound selected from the group consisting of (meth)acrylic acid and salts thereof, and acrylamide.

As the monomer for obtaining the polymer particles, a monomer other than the above-mentioned ethylenically unsaturated monomers may be used. It is possible to use such monomers, for example, by mixing with an aqueous solution including the above-mentioned ethylenically unsaturated monomers. The use amount of ethylenically unsaturated monomer may be 70 mol% to 100 mol% with respect to the total amount of monomers. The ratio of (meth)acrylic acid and salts thereof may be 70 mol% to 100 mol% with respect to the total amount of monomers.

Although the crosslinking occurs due to self-crosslinking during polymerization, the crosslinking may be promoted by using an internal crosslinking agent. The use of an internal crosslinking agent facilitates control of the water-absorbent characteristics (water retention capacity amount and the like) of the polymer particles. An internal crosslinking agent is usually added to the reaction solution during the polymerization reaction.

The polymer particles may be crosslinked in the vicinity of the surface (surface crosslinking). The polymer particles may be formed only of a crosslinking polymer or may further include a component selected from, for example, a gel stabilizer, a metal chelating agent, a flowability improver (lubricant), or the like. These components may be arranged inside the polymer particles, on the surface of the polymer particles, or both.

The shape of the polymer particles is not particularly limited and may be, for example, a substantially spherical shape, a crushed shape, or a granular shape, or may be a shape in which primary particles having these shapes are aggregated. The median particle diameter of the polymer particles may be 100 $\mu$m to 800 $\mu$m, 150 $\mu$m to 700 $\mu$m, 200 $\mu$m to 600 $\mu$m, or 250 $\mu$m to 500 $\mu$m.

The coating layer is able to impede the polymer particles from coming into contact with the liquid to be absorbed. The coating layer may coat all or a part of the surface of the polymer particles, but coats at least a part of the surface of the polymer particles. The coating layer may have a single-layer structure or may have a multilayer structure having two or more layers.

It is possible to form the coating layer by mixing polymer particles and a coating material. From the viewpoint of exhibiting a suitably practical performance when the coated resin particles are used in sanitary materials such as disposable diapers, sanitary products, and portable toilets, the ratio of the coating material mixed with the polymer particles (the ratio of the coating material) may be 0.1% by mass or more, 0.2% by mass or more, 0.5% by mass or more, 1.0% by mass or more, 1.5% by mass or more, 2.0% by mass or more, 5% by mass or more, 10% by mass or more, or 15% by mass or more. The ratio of the coating material may be 50% by mass or less, 40% by mass or less, 30% by mass or less, 25% by mass or less, or 20% by mass or less. By changing, as appropriate, the ratio of the coating material, it is possible to change the water absorption rate of the coated resin particles.

The coating material includes a polymer component. The glass transition temperature (Tg) of the polymer component may be, for example, 50°C to 100°C, 52°C to 90°C, 55°C to 80°C, or 60°C to 70°C. The 10% weight-loss temperature (10% Td) of the polymer component may be, for example, 200°C to 500°C, 250°C to 490°C, 280°C to 480°C, 300°C to 470°C, or 350°C to 460°C. The glass transition temperature (Tg) and 10% weight-loss temperature (10% Td) may be within the above-mentioned ranges from the viewpoints of the easy formation of a coating layer having a uniform thickness and facilitating control such that the water absorption rate is slowed down.

The polymer component may or may not be water-soluble (or may be poorly water-soluble). The polymer component may include a water-soluble component and may include a poorly water-soluble component. "Water-soluble" means a solubility of 1 g or more (for example, 1 g to 150 g) in 100 g of ion-exchanged water at 25°C. "Poorly water-soluble" means a solubility of less than 1 g in 100 g of ion-exchanged water at 25°C.

The water-soluble component may include a compound having a hydrophilic group. Examples of hydrophilic groups include anionic groups, cationic groups, amphoteric groups, and nonionic groups. Examples of anionic groups include carboxyl groups, sulfonic acid groups, and phosphate groups. Examples of cationic groups include amino groups, imino groups, and quaternary ammonium groups. Examples of amphoteric groups include carbobetaine groups, sulfobetaine groups, and phosphobetaine groups. Examples of nonionic groups include hydroxyl groups, amide groups, pyrrolidone groups, lactam groups, alkoxy groups, and (poly)oxyalkylene groups.

Examples of compounds having hydroxyl groups include polyvinyl alcohol and the like. Examples of compounds having an amide group include polyacrylamide and the like. Examples of compounds having a (poly)oxyalkylene group include polyalkylene oxides (for example, polyethylene oxide), polyalkylene glycols (for example, polyethylene glycol), and the like.

Examples of poorly water-soluble components include polyoxyalkylene alkyl ethers such as polyoxyethylene oleyl ether, polyoxyethylene lauryl ether, and polyoxyethylene stearyl ether; polyesters such as polyethylene terephthalate, polybutylene terephthalate, polyethylene naphthalate, and polybutylene naphthalate; polyamides such as nylon 6 and nylon 66; polyolefins such as polyethylene, polypropylene, ethylene-butene copolymers, and ethylene-propylene copolymers; polystyrenes such as poly-$\alpha$-methylstyrene and syndiotactic polystyrene; polycarbonates such as polyhexamethylene carbonate; poly(meth)acrylates such as trimethylolpropane tri(meth)acrylate, pentaerythritol tri(meth)acrylate, and dipentaerythritol hexa(meth)acrylate; polyalkyl(meth)acrylates such as polymethyl(meth)acrylate; polyacetals such as polyoxymethylene, polyacetaldehyde, polypropionaldehyde, and polybutylaldehyde; vinyl halide polymers such as polyvinyl chloride and polyvinyl fluoride; polyvinylidene fluoride; and polysiloxane.

The polymer component may include a polymer having an ethylenically unsaturated monomer as a monomer unit (a polymer having a structural unit derived from an ethylenically unsaturated monomer). Examples of ethylenically unsaturated monomers include (meth)acrylic acid and salts thereof, (meth)acrylic acid esters (methyl(meth)acrylate, ethyl(meth)acrylate, 2-hydroxyethyl(meth)acrylate, 2-(diethylamino) ethyl(meth)acrylate, 2-(diethylamino) propyl(meth)acrylate, and the like), (meth)acrylamide-based monomers ((meth)acrylamide, N-isopropyl(meth)acrylamide, 2-(meth)acrylamide-2-methylpropanesulfonic acid and salts thereof, N,N-dimethyl(meth)acrylamide, N-methylol(meth)acrylamide, diethylaminopropyl(meth)acrylamide, and the like), polyethylene glycol mono(meth)acrylate, and the like.

As polymer components, chain polymerization reaction products such as poly(meth)acrylic acid, poly(meth)acrylamide, polyvinyl alcohol, polyalkylene oxide, and polyalkylene glycol; sequential polymerization reaction products such as phenol resins (for example, condensates of a phenolic compound and aldehyde), polyester, polyamide, and polycarbonate, and the like may be used.

A water-insoluble organic compound may be acid-modified. The water-insoluble organic compound may be acid-modified using, for example, an acid anhydride (maleic anhydride, succinic anhydride, phthalic anhydride, and the like).

From the viewpoint of easily attaining a slow water absorption rate, the polymer components may include at least one selected from the group consisting of polyvinyl alcohol, polyacrylamide, polyalkylene oxide, polyalkylene glycol, polyoxyalkylene alkyl ether, polyalkyl(meth)acrylate, polyolefin, copolymers of an olefin and a water-soluble ethylenically unsaturated monomer, and copolymers of the monomers forming these polymers and preferably includes at least one selected from the group consisting of polyvinyl alcohol, polyalkylene glycol, polyalkyl(meth)acrylate, and copolymers of an olefin and a water-soluble ethylenically unsaturated monomer. In a case where the polymer component includes a copolymer of an olefin and a water-insoluble ethylenically unsaturated monomer, it is preferable to use a mixed composition in combination with polyalkylene glycol as the polymer component and more preferable to use a mixed composition in combination with polyethylene glycol as the polymer component.

As the olefin, which is a constituent unit of the polyolefin, and the olefin, which is a constituent unit of the olefin and the water-soluble ethylenically unsaturated monomer copolymer, it is preferable to use, for example, at least one selected from ethylene, propylene, and butene and more preferable to use ethylene. As the water-soluble ethylenically unsaturated monomer, it is possible to use the compounds listed above as constituent materials of the above-mentioned polymer particles and (meth)acrylic acid and/or salts thereof are preferably used.

It is possible to form the coating layer using, for example, a liquid-form or gel-form coating material (simply referred to below as the "coating liquid"). It is also possible to obtain the coating liquid, for example, by melting the coating material, or by dissolving or dispersing the coating material in any solvent or dispersion medium. The coating liquid is preferably obtained by dissolving or dispersing the coating material in any solvent or dispersion medium, since a coating layer having a uniform thickness is easily formed.

Examples of solvents or dispersion media include water, hydrophilic compounds, mixtures of water and hydrophilic compounds, hydrocarbon compounds, and the like. A hydrophilic compound is a compound that dissolves substantially uniformly in water. Examples of hydrophilic compounds include alcohols such as methanol and isopropyl alcohol; glycols such as ethylene glycol; cellosolves such as methyl cellosolve and ethyl cellosolve; ketones such as acetone and methyl ethyl ketone; esters such as ethyl acetate; ethers such as tetrahydrofurane; and the like. Examples of hydrocarbon compounds include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; aromatic hydrocarbons such as benzene, toluene, and xylene; and the like. The above may be used alone or in a combination of two or more.

The concentration of the coating material in the coating liquid is not particularly limited and is able to be adjusted, as appropriate, in consideration of the amount of water-absorbent resin particles to be coated in order to obtain a coating layer with the desired thickness, but may be, for example, 1% by mass to 50% by mass, 3% by mass to 30% by mass, or 5% by mass to 20% by mass.

Since it is easy to form a coating layer having a uniform thickness, as methods for producing coated resin particles, (1) a method for adding a coating liquid to a hydrocarbon dispersion medium in which polymer particles are dispersed, (2) a method for adding a coating liquid and polymer particles to a hydrocarbon dispersion medium substantially simultaneously, or (3) a method for bringing polymer particles in a dry state into contact with a coating liquid may be used and method (3) is more preferable.

Examples of the above-mentioned method (3) include (3-1) a method using an eggplant flask, (3-2) a method using a sprayer, and (3-3) a method using various granulators.

In method (3-1), the coating liquid is injected into the eggplant flask and then the polymer particles are injected. The eggplant flask is attached to an evaporator, heated while being rotated, and the solvent or dispersion medium included in the coating liquid is distilled off under reduced pressure conditions to obtain coated resin particles. The heating when distilling off the solvent or dispersion medium included in the coating liquid and the heat treatment described below may be carried out separately (that is, the heat treatment is carried out after obtaining the coated resin particles and then transferring the particles to another apparatus as necessary) or may be carried out at the same time (that is, the heating when distilling off the solvent or dispersion medium included in the coating liquid is carried out in the temperature range of the heat treatment described below). Since it is easy to form a coating layer having a uniform thickness, it is preferable to carry out the heating and heat treatment step when obtaining the coated resin particles separately. In such a case, the heating when obtaining the coated resin particles is preferably at the Tg or lower of the polymer component included in the coating layer.

In method (3-2), polymer particles are added to a separable flask provided with a stirrer blade and stirred and the coating liquid is sprayed onto the polymer particles wound up by stirring with the stirrer blade. It is possible to perform the spraying of the coating liquid using, for example, a two-fluid nozzle. Since it is possible to expect uniform coating, the coating liquid is desirably sprayed in the form of a mist using a stream of an inert gas such as nitrogen. Thereafter, the coated resin particles are obtained by distilling off the solvent or dispersion medium included in the coating liquid by heating. For the same reason as in the above-mentioned (3-1), the heating when distilling off the solvent or dispersion medium included in the coating liquid and the heat treatment described below may be carried out separately or may be carried out at the same time, but are preferably carried out separately and more preferably carried out separately with the heating when obtaining the coated resin particles being at the Tg or lower of the polymer component included in the coating layer.

Examples of the granulators used in method (3-3) include tumbling granulators, stirring granulators, fluidized bed granulators, and the like.

In a case where a tumbling granulator is used, an inclined shallow circular container attached to the tumbling granulator is rotated in advance and polymer particles are supplied to the circular container together with adding an appropriate amount of the coating liquid. As a result, the solvent or dispersion medium included in the coating liquid forms a coating layer on the surface of the tumbling polymer particles while some of the polymer particles undergo aggregation. It is possible to perform the step for adding the polymer particles and the coating liquid a plurality of times as necessary. Thereafter, the coating liquid is heated by any heating means (for example, hot air or heating of the circular container itself) to distill off the solvent or dispersion medium and obtain coated resin particles.

In a case of using a stirring granulator, the polymer particles are injected into a mixer attached to the stirring granulator and mixed by stirring together with adding the coating liquid. As a result, the solvent or dispersion medium included in the coating liquid forms a coating layer on the surface of the polymer particles being stirred while some of the polymer particles undergo aggregation. It is possible to perform the step for adding the polymer particles and the coating liquid a plurality of times as necessary. Thereafter, the coating liquid is heated by any heating means (for example, hot air or heating of the mixer) to distill off the solvent or dispersion medium and obtain coated resin particles.

In a case of using a fluidized bed granulator, first, the water-absorbent resin particles are injected into a container, which is attached to the fluidized bed granulator and in which it is possible to send out hot air from the lower part, and the polymer particles are preliminarily fluidized. Thereafter, when the coating liquid is scattered from a nozzle attached to the container, the solvent or dispersion medium included in the coating liquid forms a coating layer on the surface of the polymer particles being stirred while some of the polymer particles undergo aggregation. It is possible to perform the scattering of the coating liquid a plurality of times as necessary.

By heating the coating liquid using hot air, the solvent or dispersion medium is distilled off and the coated resin particles are obtained. Even in a case of using a tumbling granulator, a stirring granulator, or a fluidized bed granulator, for the same reason as in the above-mentioned (3-1), the heating when distilling off the solvent or dispersion medium included in the coating liquid and the heat treatment described below may be carried out separately or may be carried out at the same time, but are preferably carried out separately and more preferably carried out separately with the heating when obtaining the coated resin particles being at the Tg or lower of the polymer component included in the coating layer.

By heat-treating the coated resin particles at a temperature higher than the Tg of the polymer component included in the coating layer and lower than 10% Td, it is possible to obtain the water-absorbent resin particles according to the present embodiment. The temperature for the heat treatment of the coated resin particles is preferably a temperature higher than the Tg of the polymer component by 5°C to 180°C, more preferably a temperature higher than the Tg of the polymer component by 8°C to 160°C, even more preferably a temperature higher than the Tg of the polymer component by 10°C to 155°C, and yet more preferably a temperature higher than the Tg of the polymer component by 20°C to 150°C.

The apparatus used for heat-treating the coated resin particles is not particularly limited and examples thereof include a hot air-type dryer, a conductive heat transfer dryer, a radiation heat transfer dryer such as an infrared dryer, a hot air heat transfer dryer, and a dielectric heating dryer such as a microwave dryer.

**Examples**

A more specific explanation will be given below of the present invention with reference to Examples. However, the present invention is not limited to these Examples.

<Production of Polymer Film>

[Production Example A-1]

1.61 g of polyether polyol (AGC Inc., product name: EXCENOL550SO) and 4.85 g of acetone were added to a beaker with an inner diameter of 6.2 cm and having an inner surface coated with a fluorine resin and stirred to obtain a polyol solution. 1.39 g of tolylene-2,4-diisocyanate and 4.17 g of acetone were added to a 30 mL sample bottle and stirred to obtain an isocyanate solution. The isocyanate solution was added to the polyol solution while stirring and left to stand at room temperature for 18 hours to obtain a polyurethane gel. The polyurethane gel was heated to 40°C with a hot air dryer (ADVANTEC, FV-320) and dried until the weight of the polymer film reached 3.28 g to produce a polyurethane polymer film (A-1).

[Production Example A-2]

The polymer film (A-1) was heat treated for 30 minutes using a hot air dryer set at 140°C to produce a polymer film (A-2).

[Production Example A-3]

The polymer film (A-1) was heat treated for 30 minutes using a hot air dryer set at 200°C to produce a polymer film (A-3).

[Production Example B-1]

1.38 g of polyether polyol (AGC Inc., product name: EXCENOL750ED) and 4.13 g of acetone were added to a beaker with an inner diameter of 6.2 cm and having an inner surface coated with a fluorine resin and stirred to obtain a polyol solution. 1.62 g of tolylene-2,4-diisocyanate and 4.86 g of acetone were added to a 30 mL sample bottle and stirred to obtain an isocyanate solution. The isocyanate solution was added to the polyol solution while stirring and left to stand at room temperature for 18 hours to obtain a polyurethane gel. The polyurethane gel was heated to 40°C using a hot air dryer and dried until the weight of the polymer film reached 3.31 g to produce a polyurethane polymer film (B-1).

[Production Example B-2]

The polymer film (B-1) was heat treated for 30 minutes using a hot air dryer set at 140°C to produce a polymer film (B-2).

[Production Example C-1]

2.00 g of styrene-butadiene rubber (SBR, JSR Corp., product name: JSR SL552) and 38.00 g of heptane were added to a 50 mL sample bottle and immersed in a hot water bath heated to 50°C to completely dissolve the SBR and produce an SBR solution. 20.00 g of the SBR solution was added to a beaker with an inner diameter of 6.2 cm and having an inner surface coated with a fluorine resin and covered with aluminum foil as a lid. The aluminum foil was perforated, heated to 40°C using a hot air dryer, and dried until the weight of the polymer film reached 1.09 g to produce an SBR polymer film (C-1).

[Production Example C-2]

The polymer film (C-1) was heat treated for 30 minutes using a hot air dryer set at 140°C to produce a polymer film (C-2).

[Production Example D-1]

4.00 g of ethylene-sodium acrylate copolymer (a 25% water-dispersed emulsion of P(E-AANa), Sumitomo Seika Chemicals Co., Ltd., product name: ZAIKTHENE N) was added to a beaker with an inner diameter of 6.2 cm and having an inner surface coated with a fluorine resin and covered with aluminum foil as a lid. The aluminum foil was perforated, heated to 40°C using a hot air dryer, and dried until the weight of the polymer film reached 1.04 g to produce a P(E-AANa) polymer film (D-1).

[Production Example D-2]

The polymer film (D-1) was heat treated for 30 minutes using a hot air dryer set at 50°C to produce a polymer film (D-2).

[Production Example D-3]

The polymer film (D-1) was heat treated for 30 minutes using a hot air dryer set at 70°C to produce a polymer film (D-3).

[Production Example D-4]

The polymer film (D-1) was heat treated for 30 minutes using a hot air dryer set at 80°C to produce a film (D-4).

[Production Example D-5]

The polymer film (D-1) was heat treated for 30 minutes using a hot air dryer set at 100°C to produce a polymer film (D-5).

[Production Example D-6]

The polymer film (D-1) was heat treated for 30 minutes using a hot air dryer set at 140°C to produce a polymer film (D-6).

[Production Example D-7]

The polymer film (D-1) was heat treated for 30 minutes using a hot air dryer set at 200°C to produce a polymer film (D-7).

[Production Example E-1]

2.00 g of polyvinyl alcohol (PVA, Mitsubishi Chemical Corp., product name: GOHSENOL NL-05) and 18.00 g of ion-exchanged water were added to a 50 mL sample bottle and immersed in a hot water bath heated to 80°C to completely dissolve the PVA and produce a PVA solution. 10.00 g of the PVA solution was added to a beaker with an inner diameter of 6.2 cm and having an inner surface coated with a fluorine resin and covered with aluminum foil as a lid. The aluminum foil was perforated, heated to 40°C using a hot air dryer (ADVANTEC, FV-320), and dried until the weight of the polymer film reached 1.09 g to produce a PVA polymer film (E-1).

[Production Example E-2]

The polymer film (E-1) was heat treated for 30 minutes using a hot air dryer set at 140°C to produce a polymer film (E-2).

[Production Example F-1]

0.10 g of polyethylene glycol (PEG, Fujifilm Wako Pure Chemical Industries, Ltd., product name: polyethylene glycol 6000) and 0.90 g of ion-exchanged water were added to a 50 mL sample bottle and completely dissolved to produce a PEG solution. 8.00 g of a 25% water-dispersed emulsion of P(E-AANa) was added to the PEG solution and stirred to produce a P(E-AANa)/PEG solution. 4.50 g of the P(E-AANa)/PEG solution was added to a beaker with an inner diameter of 6.2 cm and having an inner surface coated with a fluorine resin and covered with aluminum foil as a lid. The aluminum foil was perforated, heated to 40°C using a hot air dryer, and dried until the weight of the polymer film reached 1.08 g to produce a P(E-AANa)/PEG polymer film (F-1).

[Production Example F-2]

The polymer film (F-1) was heat treated for 30 minutes using a hot air dryer set at 70°C to produce a polymer film (F-2).

[Production Example F-3]

The polymer film (F-1) was heat treated for 30 minutes using a hot air dryer set at 80°C to produce a polymer film (F-3).

[Production Example G-1]

2.00 g of polymethyl methacrylate (PMMA, Fujifilm Wako Pure Chemical Industries, Ltd.) and 18.00 g of tetrahydrofuran were added to a 50 mL sample bottle and stirred with a magnetic stirrer until the PMMA was completely dissolved to produce a PMMA solution. 10.00 g of the PMMA solution was added to a beaker with an inner diameter of 6.2 cm and having an inner surface coated with a fluorine resin and left to stand at room temperature for 18 hours. Thereafter, the result was heated to 40°C using a hot air dryer, and dried until the weight of the polymer film reached 1.10 g to produce a PMMA polymer film (G-1).

[Production Example G-2]

The polymer film (G-1) was heat treated for 30 minutes using a hot air dryer set at 140°C to produce a polymer film (G-2).
The glass transition temperature and 10% weight-loss temperature were measured for the polymer films before heat treatment. The elongation rate was measured for the polymer films before and after the heat treatment and the amount of change before and after the heat treatment was calculated. Table 1 shows the results.

<Glass Transition Temperature (Tg)>

3.0 mg of the polymer film was sealed in an Al-sealed sample container (Hitachi High-Tech Science Co., Ltd., GCA-0017), an empty Al-sealed sample container was used as a reference, and a high-sensitivity differential scanning calorimeter (Yamato Scientific Co., Ltd., DSC7020) was used to measure the Tg of the polymer film. For the measurement, two cycles of DSC measurement were repeated from -100°C to 200°C at a temperature rising rate/ temperature falling rate of 10°C/min. Tg was calculated from the change from the baseline.

<10% Weight-Loss Temperature (10% Td)>

3.0 mg of the polymer film was sealed in an Al-sealed sample container and the thermogravimetric loss of the polymer film was measured using a high-temperature differential thermogravimetric simultaneous measurement device (Hitachi High-Tech Science Co., Ltd., TG-DTA 7220). The measurement was performed under the conditions of nitrogen flow rate: 200 mL/min, temperature rising rate: 20°C/min, measurement temperature: 30°C to 500°C, and holding time: 5 minutes.

<Tensile Test>

Using a dumbbell cutter (Dumbbell Co., Ltd., SDMP-1000), a test piece having dimensions conforming to JIS K 6251-7 was cut from the polymer film. The test piece had one end and another end having a width of 6 mm and a linear intermediate portion (length: 12 mm) having a width of 2 mm connecting the one end and the other end and the total length of the test piece was 35 mm. Atensile test (JIS K 6251, jig: SCG-1kNA, tension rate: 200 mm/min) was performed on this test piece using a desktop precision universal testing machine (manufactured by Shimadzu Corp., Autograph AGS-X). The elongation rate [%] of the polymer film was calculated from the following equation and the amount of change in the elongation rate before and after the heat treatment was determined.

$$\text{Elongation rate [\%]} = (\text{Total length of intermediate portion at break/Total initial length of intermediate portion (12 mm))} \times 100$$

[Table 1]

| Polymer film | Polymer component | Tg (°C) | 10%Td (°C) | Heat treatment (°C) | Elongation rate (%) | Amount of change (%) |
|---|---|---|---|---|---|---|
| A-1 | Polyurethane | 55 | 271 | - | 35 | - |
| A-2 | Polyurethane | - | - | 140 | 12 | -23 |
| A-3 | Polyurethane | - | - | 200 | 16 | -19 |
| B-1 | Polyurethane | 103 | 270 | - | 9 | - |
| B-2 | Polyurethane | - | - | 140 | 6 | -3 |
| C-1 | SBR | 49 | 407 | - | 232 | - |
| C-2 | SBR | - | - | 140 | 204 | -28 |
| D-1 | P(E-AANa) | 56 | 430 | - | 163 | - |
| D-2 | P(E-AANa) | - | - | 50 | 163 | 0 |
| D-3 | P(E-AANa) | - | - | 70 | 127 | -36 |
| D-4 | P(E-AANa) | - | - | 80 | 271 | +108 |
| D-5 | P(E-AANa) | - | - | 100 | 96 | -67 |
| D-6 | P(E-AANa) | - | - | 140 | 91 | -73 |
| D-7 | P(E-AANa) | - | - | 200 | 36 | -127 |
| E-1 | PVA | 66 | 292 | - | 298 | - |
| E-2 | PVA | - | - | 140 | 19 | -279 |
| F-1 | P(E-AANa)/PEG | 56 | 473 | - | 70 | - |
| F-2 | P(E-AANa)/PEG | - | - | 70 | 27 | -43 |
| F-3 | P(E-AANa)/PEG | - | - | 80 | 125 | +55 |
| G-1 | PMMA | 73 | 368 | - | 67 | - |
| G-2 | PMMA | - | - | 140 | 6 | -61 |

<Production of Particles for Evaluation>

[Comparative Example 1]

A round-bottomed cylindrical separable flask having an inner diameter of 11 cm and a volume of 2 L and provided with a reflux cooling device, a dropping funnel, a nitrogen gas introduction tube, and a stirrer (stirrer blades having two stages of four inclined paddle blades with a blade diameter of 5 cm) was prepared. 293 g of n-heptane and 0.736 g of a maleic anhydride-modified ethylene-propylene copolymer (dispersant, Mitsui Chemicals, Inc., Hi-Wax 1105A) were added to the flask to obtain a mixture. The mixture was heated to 80°C while stirring at a rotation speed of 300 rpm to dissolve the dispersant and then the mixture was cooled to 55°C.

Subsequently, 92.0 g of an 80.5% by mass acrylic acid aqueous solution (acrylic acid: 1.03 mol) was put into a triangular flask having a volume of 500 mL. Subsequently, while cooling from the outside, 102.2 g of a 30% by mass sodium hydroxide aqueous solution was added dropwise to neutralize 75 mol% of the acrylic acid. Next, 0.092 g of hydroxyethyl cellulose (Sumitomo Seika Chemicals Co., Ltd., HEC AW-15F) as a thickener, 0.0736 g (0.272 mmol) of potassium persulfate as a water-soluble radical polymerization initiator, 0.0101 g (0.0580 mmol) of ethylene glycol diglycidyl ether as an internal crosslinking agent, and 32.85 g of ion-exchanged water were added and then dissolved to prepare a first stage aqueous solution.

After adding the above-mentioned first stage aqueous solution to the above-mentioned separable flask, stirring was carried out for 10 minutes. Thereafter, a surfactant solution obtained by dissolving 0.736 g of sucrose stearate (surfactant, manufactured by Mitsubishi Chemical Foods Co., Ltd., Ryoto Sugar Ester S-370, HLB: 3) in 6.62 g of n-heptane was added to the separable flask to obtain a reaction solution. Then, while stirring the reaction solution at a rotation speed of 550 rpm, the inside of the system was sufficiently replaced with nitrogen. Thereafter, the separable flask was immersed in a water bath at 70°C to heat the reaction solution and a polymerization reaction was carried out for 10 minutes to obtain a first stage polymerization slurry solution.

Subsequently, 128.8 g of an 80.5% by mass acrylic acid aqueous solution (acrylic acid: 1.44 mol) was put into another triangular flask having a volume of 500 mL. Subsequently, while cooling from the outside, 143.1 g of a 30% by mass sodium hydroxide aqueous solution was added dropwise to neutralize 75 mol% of acrylic acid. Thereafter, 0.1030 g (0.3810 mmol) of potassium persulfate, 0.0116 g (0.0666 mmol) of ethylene glycol diglycidyl ether, and 0.63 g of ion-exchanged water were added and then dissolved to prepare a second stage aqueous solution.

While stirring at a rotation speed of 1000 rpm, the first stage polymerization slurry solution in the above-mentioned separable flask was cooled to 25°C and the total amount of the second stage aqueous solution was added to the first stage polymerization slurry solution to obtain a reaction solution. Then, while stirring the reaction solution, the inside of the system was sufficiently replaced with nitrogen. Thereafter, the separable flask was immersed in a water bath at 70°C to heat the reaction solution and a second stage polymerization reaction was carried out for 5 minutes to obtain a second stage polymerization slurry solution.

The second stage polymerization slurry solution was heated in an oil bath at 125°C and 267 g of water was extracted to the outside of the system by azeotropic distillation of n-heptane and water while refluxing the n-heptane. Subsequently, 0.0884 g (0.5075 mmol) of ethylene glycol diglycidyl ether was added as a surface crosslinking agent and held at 83°C for 2 hours to obtain a dispersion liquid of resin particles having crosslinked surfaces.

Thereafter, the dispersion liquid of resin particles after the above-mentioned surface-crosslinking was heated in an oil bath at 125°C to evaporate the n-heptane and dried to obtain a dried product. The dried product was passed through a sieve having openings of 850 μm to obtain 232.8 g of polymer particles (1) in the form of aggregated spherical particles. The polymer particles (1) were used as particles for evaluation.

[Comparative Example 2]

1.5 g of the polymer particles (1) were scattered in a metal petri dish having a diameter of 5 cm, on which aluminum foil was used as a lid. The aluminum foil was perforated and a heat treatment was carried out for 30 minutes using a hot air dryer (ADVANTEC, FV-320) set at 80°C to obtain polymer particles (2). The polymer particles (2) were used as particles for evaluation.

[Comparative Example 3]

The polymer particles (1) were heat treated in the same manner as in Comparative Example 2, except that the temperature of the hot air dryer was changed to 140°C to obtain polymer particles (3). The polymer particles (3) were used as particles for evaluation.

[Comparative Example 4]

A round-bottomed cylindrical separable flask having an inner diameter of 110 mm and an internal volume of 2 L and provided with a reflux cooling device, a dropping funnel, a nitrogen gas introduction tube, and a stirrer (stirrer blades having two stages of four inclined paddle blades having a blade diameter of 50 mm) was prepared. 40 g of the polymer particles (1) and 480 g of n-heptane were mixed in this separable flask.

A polyol aqueous solution in which 0.4 g of polyether polyol (EXCENOL550SO) and 7.6 g of ion-exchanged water were mixed was added to a separable flask and then stirred at room temperature for 30 minutes. Thereafter, after adding an isocyanate solution in which 0.34 g of tolylene-2,4-diisocyanate and 3.06 g of acetone were mixed, the result was stirred at room temperature for 60 minutes to allow the sequential polymerization reaction to proceed on the surfaces of the polymer particles and obtain a reaction product. Subsequently, the n-heptane was removed by evaporation by heating the reaction product in an oil bath at 125°C. Then, by passing the result through a sieve having openings of 850 μm, 37.3 g of coated resin particles (1) (median particle diameter: 350 μm) provided with polymer particles and a coating layer (polyurethane) were obtained. The coated resin particles (1) were used as particles for evaluation. The coated resin particles (1) have a coating layer corresponding to the polymer film (A-1).

[Comparative Example 5]

The coated resin particles (1) were heat treated in the same manner as in Comparative Example 2 except that the setting temperature of the hot air dryer was changed to 140°C to obtain water-absorbent resin particles having a coating layer corresponding to the polymer film (A-2).

[Comparative Example 6]

The coated resin particles (1) were heat treated in the same manner as in Comparative Example 2 except that the setting temperature of the hot air dryer was changed to 200°C to obtain water-absorbent resin particles having a coating layer corresponding to the polymer film (A-3).

[Comparative Example 7]

38.2 g of coated resin particles (2) (median particle diameter: 349 $\mu$m) provided with polymer particles and a coating layer (polyurethane) were obtained with the same procedure as in the production of coated resin particles (1) except for using a polyol aqueous solution in which 0.4 g of polyether polyol (EXCENOL750ED) and 7.6 g of ion-exchanged water were mixed and an isocyanate solution in which 0.48 g of tolylene-2,4-diisocyanate and 4.30 g of acetone were mixed. The coated resin particles (2) were used as particles for evaluation. The coated resin particles (2) have a coating layer corresponding to the polymer film (B-1).

[Comparative Example 8]

The coated resin particles (2) were heat treated in the same manner as in Comparative Example 2 except that the setting temperature of the hot air dryer was changed to 140°C to obtain water-absorbent resin particles having a coating layer corresponding to the polymer film (B-2).

[Comparative Example 9]

The polymer particles obtained by repeating the same operation as in Comparative Example 1 were classified with a comb having openings of 250 $\mu$m to obtain 500 g or more of the polymer particles (1) having a particle diameter of 250 $\mu$m to 850 $\mu$m. 25 g of SBR and 475 g of tetrahydrofuran were added to a polybeaker having an internal volume of 1 L and mixed to prepare a coating liquid.
After injecting 500.0 g of the polymer particles (1) into the container of a fluidized bed granulator, warm air at 40°C was blown from the lower part of the container. Subsequently, 500 g of the coating liquid was sprayed over 90 minutes onto the polymer particles being wound up with warm air at 40°C and then dried by blowing air at 40°C for 30 minutes. Thus, 510.4 g of coated resin particles (3) (median particle diameter: 396 $\mu$m) provided with polymer particles and a coating layer (SBR) were obtained. The coated resin particles (3) were used as particles for evaluation. The coated resin particles (3) have a coating layer corresponding to the polymer film (C-1).

[Comparative Example 10]

The coated resin particles (3) were heat treated in the same manner as in Comparative Example 2 except that the setting temperature of the hot air dryer was changed to 140°C to obtain water-absorbent resin particles having a coating layer corresponding to the polymer film (C-2).

[Comparative Example 11]

503.2 g of coated resin particles (4) (median particle diameter: 371 $\mu$m) provided with polymer particles and a coating layer (P(E-AANa)) were obtained in the same manner as in Comparative Example 9 except that the coating liquid was changed to a mixed solution of 40.0 g of a 25% water-dispersed emulsion of P(E-AANa) and 60.0 g of ion-exchanged water and the coating liquid was sprayed over 20 minutes on polymer particles wound up with warm air at 50°C and dried by blowing air at 50°C. The coated resin particles (4) were used as particles for evaluation. The coated resin particles (4) have a coating layer corresponding to the polymer film (D-1).

[Comparative Example 12]

The coated resin particles (4) were heat treated in the same manner as in Comparative Example 2 except that the setting temperature of the hot air dryer was changed to 50°C to obtain water-absorbent resin particles having a coating layer corresponding to the polymer film (D-2).

[Comparative Example 13]

502.1 g of coated resin particles (5) (median particle diameter: 349 $\mu$m) provided with polymer particles and a coating layer (P(E-AANa)) were obtained in the same manner as in Comparative Example 9 except that the coating liquid was changed to a mixed solution of 200.0 g of a 25% water-dispersed emulsion of P(E-AANa) and 300.0 g of ion-exchanged water and the coating liquid was sprayed over 90 minutes on polymer particles wound up with warm air at 50°C and dried by blowing air at 50°C. The coated resin particles (5) were used as particles for evaluation. The coated resin particles (5) have a coating layer corresponding to the polymer film (D-1).

[Comparative Example 14]

The coated resin particles (5) were heat treated in the same manner as in Comparative Example 2 except that the setting temperature of the hot air dryer was changed to 50°C to obtain water-absorbent resin particles having a coating layer corresponding to the polymer film (D-2).

[Comparative Example 15]

513.3 g of coated resin particles (6) (median particle diameter: 390 $\mu$m) provided with polymer particles and a coating layer (PVA) were obtained in the same manner as in Comparative Example 9 except that the coating liquid was changed to a mixed solution of 100.0 g of PVA, 1330 g of ion-exchanged water, and 570 g of ethanol and the coating liquid was sprayed over 360 minutes on polymer particles wound up with warm air at 80°C and dried by blowing air at 80°C. The coated resin particles (6) were used as particles for evaluation. The coated resin particles (6) have a coating layer corresponding to the polymer film (E-1).

[Comparative Example 16]

503.8 g of coated resin particles (7) (median particle diameter: 394 $\mu$m) provided with polymer particles and a coating layer (P(E-AANa)/PEG) were obtained in the same manner as in Comparative Example 9 except that the coating liquid was changed to a mixed solution of 200.0 g of a 25% water-dispersed emulsion of P(E-AANa), 2.5 g of polyethylene glycol (PEG 6000), and 297.5 g of ion-exchanged water and the coating liquid was sprayed over 90 minutes on polymer particles wound up with warm air at 50°C and dried by blowing air at 50°C. The coated resin particles (7) were used as particles for evaluation. The coated resin particles (7) have a coating layer corresponding to the polymer film (F-1).

[Comparative Example 17]

505.5 g of coated resin particles (8) (median particle diameter: 375 $\mu$m) provided with polymer particles and a coating layer (PMMA) were obtained in the same manner as in Comparative Example 9 except that the coating liquid was changed to a mixed solution of 50.0 g of PMMA and 450 g of tetrahydrofuran. The coated resin particles (8) were used as particles for evaluation. The coated resin particles (8) have a coating layer corresponding to the polymer film (G-1).

[Example 1]

The coated resin particles (4) were heat treated in the same manner as in Comparative Example 2 except that the setting temperature of the hot air dryer was changed to 70°C to obtain water-absorbent resin particles having a coating layer corresponding to the polymer film (D-3).

[Example 2]

The coated resin particles (4) were heat treated in the same manner as in Comparative Example 2 to obtain water-absorbent resin particles having a coating layer corresponding to the polymer film (D-4).

[Example 3]

The coated resin particles (4) were heat treated in the same manner as in Comparative Example 2 except that the setting temperature of the hot air dryer was changed to 100°C to obtain water-absorbent resin particles having a coating layer corresponding to the polymer film (D-5).

[Example 4]

The coated resin particles (4) were heat treated in the same manner as in Comparative Example 2 except that the setting temperature of the hot air dryer was changed to 140°C to obtain water-absorbent resin particles having a coating layer corresponding to the polymer film (D-6).

[Example 5]

The coated resin particles (4) were heat treated in the same manner as in Comparative Example 2 except that the setting temperature of the hot air dryer was changed to 200°C to obtain water-absorbent resin particles having a coating layer corresponding to the polymer film (D-7).

[Example 6]

The coated resin particles (5) were heat treated in the same manner as in Comparative Example 2 except that the setting temperature of the hot air dryer was changed to 70°C to obtain water-absorbent resin particles having a coating layer corresponding to the polymer film (D-3).

[Example 7]

The coated resin particles (5) were heat treated in the same manner as in Comparative Example 2 to obtain water-absorbent resin particles having a coating layer corresponding to the polymer film (D-4).

[Example 8]

The coated resin particles (5) were heat treated in the same manner as in Comparative Example 2 except that the setting temperature of the hot air dryer was changed to 100°C to obtain water-absorbent resin particles having a coating layer corresponding to the polymer film (D-5).

[Example 9]

The coated resin particles (5) were heat treated in the same manner as in Comparative Example 2 except that the setting temperature of the hot air dryer was changed to 140°C to obtain water-absorbent resin particles having a coating layer corresponding to the polymer film (D-6).

[Example 10]

The coated resin particles (5) were heat treated in the same manner as in Comparative Example 2 except that the setting temperature of the hot air dryer was changed to 200°C to obtain water-absorbent resin particles having a coating layer corresponding to the polymer film (D-7).

[Example 11]

The coated resin particles (6) were heat treated in the same manner as in Comparative Example 2 except that the setting temperature of the hot air dryer was changed to 140°C to obtain water-absorbent resin particles having a coating layer corresponding to the polymer film (E-2).

[Example 12]

The coated resin particles (7) were heat treated in the same manner as in Comparative Example 2 except that the setting temperature of the hot air dryer was changed to 70°C to obtain water-absorbent resin particles having a coating layer corresponding to the polymer film (F-2).

[Example 13]

The coated resin particles (7) were heat treated in the same manner as in Comparative Example 2 to obtain water-absorbent resin particles having a coating layer corresponding to the polymer film (F-3).

[Example 14]

The coated resin particles (8) were heat treated in the same manner as in Comparative Example 2 except that the setting temperature of the hot air dryer was changed to 140°C to obtain water-absorbent resin particles having a coating layer corresponding to the polymer film (G-2).

[Evaluation of Water Absorption Rate]

0.200 g of particles for evaluation were precisely weighed and laid in layers on the bottom of an acrylic cylinder having an inner diameter of 2.0 cm and a depth of 8.0 cm to form a particle layer and then the height $H_0$ of the particle layer was measured at 25°C. Thereafter, 20 g of physiological saline was poured in from the upper part of the acrylic cylinder. The measurement was started at the time when the total amount of the physiological saline was put in, the height $H_3$ of the particle layer was read after 3 minutes, and the water absorption rate was calculated from the following equation. Table 2 shows the results of the particles for evaluation of the Comparative Examples and Table 3 shows the results of the particles for evaluation of the Examples. A lower water absorption rate (cm) value indicates a slower water absorption rate.

$$\text{Water absorption rate (cm)} = H_3 - H_0$$

[Table 2]

| Comparative Examples | | Coating layer | | Temperature (°C) | Water absorption rate (cm) | Amount of change (cm) |
|---|---|---|---|---|---|---|
| | | Polymer component | Added amount (% by mass) | | | |
| 1 | Polymer particles (1) | - | - | - | 5.1 | - |
| 2 | Polymer particles (2) | - | - | 80 | 4.9 | -0.2 |
| 3 | Polymer particles (3) | - | - | 140 | 5.4 | 0.3 |
| 4 | Coated resin particles (1) | Polyurethane | 2 | - | 4.7 | - |
| 5 | Coated resin particles (1) | Polyurethane | 2 | 140 | 4.6 | -0.1 |
| 6 | Coated resin particles (1) | Polyurethane | 2 | 200 | 4.2 | -0.5 |
| 7 | Coated resin particles (2) | Polyurethane | 2 | - | 2.2 | - |
| 8 | Coated resin particles (2) | Polyurethane | 2 | 140 | 2.3 | 0.1 |
| 9 | Coated resin particles (3) | SBR | 5 | - | 4.4 | - |
| 10 | Coated resin particles (3) | SBR | 5 | 140 | 4.3 | -0.1 |
| 11 | Coated resin particles (4) | P(E-AANa) | 2 | - | 4.9 | - |
| 12 | Coated resin particles (4) | P(E-AANa) | 2 | 50 | 4.7 | -0.2 |

(continued)

| Comparative Examples | | Coating layer | | Temperature (°C) | Water absorption rate (cm) | Amount of change (cm) |
| --- | --- | --- | --- | --- | --- | --- |
| | | Polymer component | Added amount (% by mass) | | | |
| 13 | Coated resin particles (5) | P(E-AANa) | 10 | - | 4.3 | - |
| 14 | Coated resin particles (5) | P(E-AANa) | 10 | 50 | 3.7 | -0.6 |
| 15 | Coated resin particles (6) | PVA | 20 | - | 2.7 | - |
| 16 | Coated resin particles (7) | P(E-AANa) /PEG | 10/0.5 | - | 4.6 | - |
| 17 | Coated resin particles (8) | PMMA | 10 | - | 3.2 | - |

[Table 3]

| Examples | | Coating layer | | Temperature (°C) | Water absorption rate (cm) | Amount of change (cm) |
| --- | --- | --- | --- | --- | --- | --- |
| | | Polymer component | Added amount (% by mass) | | | |
| 1 | Coated resin particles (4) | P(E-AANa) | 2 | 70 | 3.7 | -1.2 |
| 2 | Coated resin particles (4) | P(E-AANa) | 2 | 80 | 3.5 | -1.4 |
| 3 | Coated resin particles (4) | P(E-AANa) | 2 | 100 | 3.4 | -1.5 |
| 4 | Coated resin particles (4) | P(E-AANa) | 2 | 140 | 2.5 | -2.4 |
| 5 | Coated resin particles (4) | P(E-AANa) | 2 | 200 | 1.9 | -3.0 |
| 6 | Coated resin particles (5) | P(E-AANa) | 10 | 70 | 0.5 | -3.8 |
| 7 | Coated resin particles (5) | P(E-AANa) | 10 | 80 | 0.6 | -3.7 |
| 8 | Coated resin particles (5) | P(E-AANa) | 10 | 100 | 1.0 | -3.3 |
| 9 | Coated resin particles (5) | P(E-AANa) | 10 | 140 | 1.1 | -3.2 |
| 10 | Coated resin particles (5) | P(E-AANa) | 10 | 200 | 1.8 | -2.5 |
| 11 | Coated resin particles (6) | PVA | 20 | 140 | 1.0 | -1.7 |
| 12 | Coated resin particles (7) | P(E-AANa) /PEG | 10/0.5 | 70 | 3.5 | -1.1 |
| 13 | Coated resin particles (7) | P(E-AANa) /PEG | 10/0.5 | 80 | 0.9 | -3.7 |

(continued)

| Examples | | Coating layer | | Temperature (°C) | Water absorption rate (cm) | Amount of change (cm) |
| --- | --- | --- | --- | --- | --- | --- |
| | | Polymer component | Added amount (% by mass) | | | |
| 14 | Coated resin particles (8) | PMMA | 10 | 140 | 2.1 | -1.1 |

## Claims

1. A method for producing water-absorbent resin particles, the method comprising:

   forming a coating layer on at least part of a surface of water-absorbent polymer particles to obtain coated resin particles; and
   heat-treating the coated resin particles, at a temperature higher than a glass transition temperature of a polymer component included in the coating layer and lower than a 10% weight-loss temperature, to obtain water-absorbent resin particles,
   wherein an absolute value for an amount of change in an elongation rate of the polymer component before and after the heat treatment is 30% or more and 500% or less.

2. The method for producing water-absorbent resin particles according to claim 1,
   wherein the temperature of the heat treatment is higher than the glass transition temperature of the polymer component by 5°C to 180°C.

3. The method for producing water-absorbent resin particles according to claim 1 or 2,
   wherein the polymer component includes at least one polymer selected from the group consisting of polyvinyl alcohol, polyacrylamide, polyalkylene oxide, polyalkylene glycol, polyoxyalkylene alkyl ether, polyalkyl(meth)acrylate, polyolefin, a copolymer of an olefin and a water-soluble ethylenically unsaturated monomer, and copolymers of monomers forming these polymers.

4. The method for producing water-absorbent resin particles according to claim 1 or 2,
   wherein the polymer component includes at least one selected from the group consisting of polyvinyl alcohol, polyalkylene glycol, polyalkyl(meth)acrylate, and a copolymer of an olefin and a water-soluble ethylenically unsaturated monomer.

5. The method for producing water-absorbent resin particles according to any one of claims 1 to 4,
   wherein the glass transition temperature of the polymer component is 50°C to 100°C.

**INTERNATIONAL SEARCH REPORT**

International application No.

**PCT/JP2022/008693**

#### A. CLASSIFICATION OF SUBJECT MATTER

*C08J 3/12*(2006.01)i
FI: C08J3/12 Z CER; C08J3/12 CEZ

According to International Patent Classification (IPC) or to both national classification and IPC

#### B. FIELDS SEARCHED

Minimum documentation searched (classification system followed by classification symbols)

C08J3/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

#### C. DOCUMENTS CONSIDERED TO BE RELEVANT

| Category* | Citation of document, with indication, where appropriate, of the relevant passages | Relevant to claim No. |
|---|---|---|
| X | JP 11-347403 A (SANYO CHEM. IND. LTD.) 21 December 1999 (1999-12-21) claims, paragraphs [0011]-[0019], examples | 1-5 |
| X | JP 2010-513631 A (BASF SE) 30 April 2010 (2010-04-30) claims, paragraphs [0023]-[0038], examples | 1-5 |
| A | JP 3-285918 A (TOKAI RUBBER IND. LTD.) 17 December 1991 (1991-12-17) entire text | 1-5 |
| A | JP 2016-508167 A (BASF SE) 17 March 2016 (2016-03-17) entire text | 1-5 |

☐ Further documents are listed in the continuation of Box C.       ☑ See patent family annex.

| | |
|---|---|
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **12 May 2022** | **24 May 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2022/008693**

| Patent document cited in search report | | | Publication date (day/month/year) | Patent family member(s) | Publication date (day/month/year) |
|---|---|---|---|---|---|
| JP | 11-347403 | A | 21 December 1999 | (Family: none) | |
| JP | 2010-513631 | A | 30 April 2010 | US 2010/0294988 A1 claims, paragraphs [0023]-[0048], examples | |
| JP | 3-285918 | A | 17 December 1991 | (Family: none) | |
| JP | 2016-508167 | A | 17 March 2016 | US 2015/0314034 A1 entire text | |

Form PCT/ISA/210 (patent family annex) (January 2015)

**EP 4 317 261 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016028117 A